# EUROPÄISCHE PATENTANMELDUNG

(11) **EP 3 150 233 A1**
(43) Veröffentlichungstag der Anmeldung: **05.04.2017**
(21) Anmeldenummer: 15187929.3
(22) Anmeldetag: 01.10.2015
(51) Int. Cl.: A61L 2/26, A61L 2/24

(54) **VERFAHREN ZUM REINIGEN UND PFLEGEN EINES ÄRZTLICHEN BEHANDLUNGSINSTRUMENTS**

(71) Anmelder: Kaltenbach & Voigt GmbH, 88400 Biberach (DE)
(72) Erfinder: Wiek, Hans-Dieter, 88454 Hochdorf (DE)
(74) Vertreter: Thun, Clemens

(57) **Zusammenfassung**

Gemäß der Erfindung ist Verfahren zum Reinigen und Pflegen eines ärztlichen, insbesondere zahnärztlichen Behandlungsinstruments vorgesehen, bei dem das Instrument mit Hilfe eines Reinigungsmittels gereinigt (I) und anschließend mittels eines ölhaltigen Pflegemittels gepflegt (V) wird. Dabei wird vor dem Zuführen des ölhaltigen Pflegemittels (V) zunächst das Reinigungsmittel in einem Spülschritt (III) im Wesentlichen vollständig entfernt. Durch das Entfernen des Reinigungsmittels lässt sich erzielen, dass das ölhaltige Pflegemittel besser wirken kann. Insbesondere lässt sich so die Gefahr verringern, dass Reste des Reinigungsmittels, die sich nach dem Reinigungsvorgang noch im Inneren des Behandlungsinstruments befinden, eine effektive Schmierung an Gleit- und Lagerstellen des Behandlungsinstruments durch das ölhaltige Pflegemittel negativ beeinträchtigen.

## Beschreibung

Die Erfindung betrifft ein Verfahren zum Reinigen und Pflegen eines ärztlichen, insbesondere zahnärztlichen Behandlungsinstruments, bei dem das Instrument mit Hilfe eines Reinigungsmittels gereinigt und anschließend mittels einem ölhaltigen Pflegemittel gepflegt wird.

Bei einem ärztlichen oder zahnärztlichen Behandlungsinstrument handelt es sich typischerweise um ein im Wesentlichen rohrförmiges Teil, das ein Arzt bzw. Zahnarzt während der Behandlung ergreift. Ein üblicherweise in der zahnmedizinischen Praxis verwendetes Behandlungsinstrument ist ein sogenanntes Bohrhandstück, das an seinem vorderen Ende ein Behandlungswerkzeug, insbesondere einen Bohrer trägt und das mit seinem hinteren Ende mittels einer Kupplung mit einem Versorgungsschlauch gekoppelt ist. Das Behandlungswerkzeug kann ausgewechselt werden und ist mithilfe eines Spannsystems gehalten.

Durch das Handstück erstrecken sich typischerweise Versorgungsleitungen für Energie zum Antrieb des Behandlungsinstruments sowie Fluidleitungen für Behandlungsmedien, beispielsweise Luft und/oder Wasser. Unterschieden wird hierbei in der Regel zwischen sogenannten Turbinen-Handstücken, bei denen zur Versorgung einer im vorderen Endbereich angeordneten Turbine Druckluft vorgesehen ist und sogenannten Motor-Handstücken, welche als Antriebseinheit einen Elektromotor aufweisen.

Zur Aufrechterhaltung der Funktion eines solchen Behandlungsinstruments bedarf es von Zeit zu Zeit einer Aufbereitung bzw. Pflege, beispielsweise von drehbar gelagerten Elementen des Instruments. Ferner führen die in der zahnärztlichen Praxis immer weiter ansteigenden Hygieneanforderungen dazu, dass Behandlungsinstrumente in regelmäßigen zeitlichen Abständen aufbereitet werden müssen.

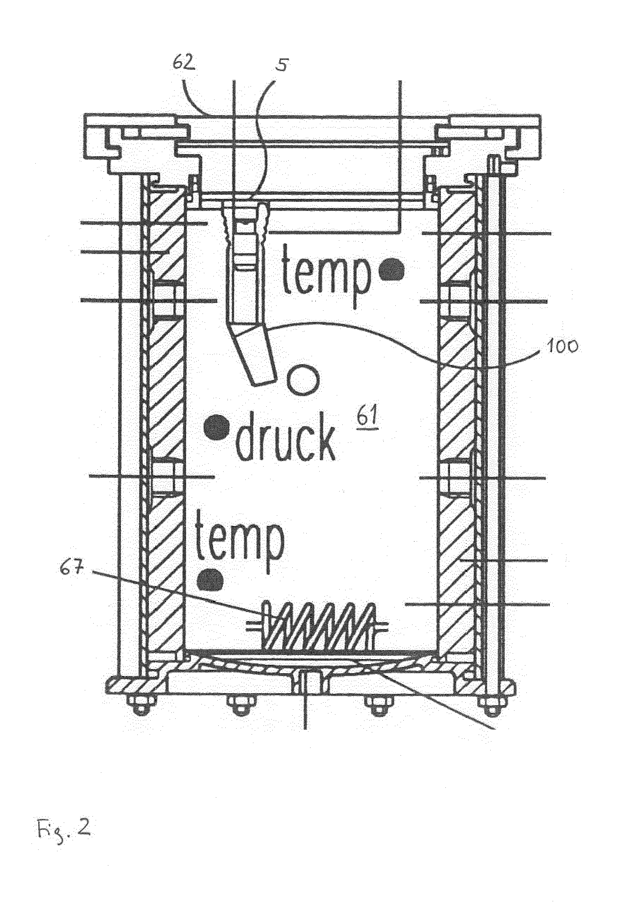

Aus der WO 2011/101396 A1 ist ein Gerät zum Aufbereiten von zahnärztlichen Behandlungsinstrumenten bekannt. Das Gerät umfasst eine, in Fig. 2 skizzierte Kammer bzw. Pflegekammer 61, in der ein aufzubereitendes Instrument 100 mit Hilfe einer Kupplung 5 angeordnet werden kann. In einem ersten Schritt wird dann ein Reinigungsmittel durch einen Innenraum des Behandlungsinstruments 100 gespült. Auf diese Weise lassen sich Proteine, Speichel und weitere organische Verschmutzungen aus dem Instrumenteninneren entfernen. In einem weiteren Schritt wird dann der Innenraum des Instruments mit einem Ölpflegemittel behandelt. Wie in Fig. 2 angedeutet, kann die Kammer 61 mit einem Deckel 62 verschließbar sein und eine Heizung 67 umfassen.

Die Praxis hat nunmehr gezeigt, dass sich ein entsprechendes Behandlungsinstrument nach einer derartigen Aufbereitung bisweilen nicht mehr einwandfrei bedienen lässt; insbesondere kann es im Fall eines Bohrhandstücks dazu kommen, dass das Spannsystem, mit dem der Bohrer gehalten ist, nur sehr schwer bedient werden kann.

Der Erfindung liegt die Aufgabe zugrunde, ein verbessertes Verfahren zum Reinigen und Pflegen eines ärztlichen, insbesondere zahnärztlichen Behandlungsinstruments anzugeben. Insbesondere soll sich durch das Verfahren die Gefahr verringern lassen, dass sich das behandelte Instrument nicht mehr einwandfrei bedienen lässt.

Diese Aufgabe wird gemäß der Erfindung mit dem in dem unabhängigen Anspruch genannten Gegenstand gelöst. Besondere Ausführungsarten der Erfindung sind in den abhängigen Ansprüchen angegeben.

Gemäß der Erfindung ist Verfahren zum Reinigen und Pflegen eines ärztlichen, insbesondere zahnärztlichen Behandlungsinstruments vorgesehen, bei dem das Instrument mit Hilfe eines Reinigungsmittels gereinigt und anschließend mittels eines ölhaltigen Pflegemittels gepflegt wird. Dabei wird vor dem Zuführen des ölhaltigen Pflegemittels zunächst das Reinigungsmittel in einem Spülschritt im Wesentlichen vollständig entfernt.

Durch das Entfernen des Reinigungsmittels lässt sich erzielen, dass das ölhaltige Pflegemittel besser wirken kann. Insbesondere lässt sich so die Gefahr verringern, dass Reste des Reinigungsmittels, die sich nach dem Reinigungsvorgang bzw. -schritt noch im Inneren des Behandlungsinstruments befinden, eine effektive Schmierung an Gleit- oder Lagerstellen des Behandlungsinstruments durch das ölhaltige Pflegemittel negativ beeinträchtigen.

Vorzugsweise wird dabei in dem Spülschritt das Instrument mit Wasser gespült. Hierdurch lässt sich das Reinigungsmittel besonders wirksam entfernen, insbesondere wenn es sich bei dem Wasser um VE-Wasser (vollentsalztes Wasser) handelt.

Vorzugsweise wird nach dem Spülschritt vor einem Zuführen des ölhaltigen Pflegemittels das Instrument mit Wasserdampf gereinigt. Durch die hierbei erfolgende Erwärmung des Instruments lässt sich insbesondere erzielen, dass das ölhaltige Pflegemittel besonders geeignet aufgetragen werden kann.

Vorzugsweise wird nach dem Zuführen des Reinigungsmittels dieses vor dem Spülschritt zunächst mittels Druckluft aus bzw. von dem Instrument wenigstens teilweise entfernt. Hierdurch lässt sich das Reinigungsmittel im folgenden Spülschritt besonders geeignet wirksam entfernen.

Vorzugsweise enthält das ölhaltige Pflegemittel einen Emulgator. Hierdurch lässt sich insbesondere eine geeignete Schmierung an Bauteilen des Behandlungsinstruments erzielen, die unter Wasser stehen oder an denen Wasserreste angelagert sind.

Die Erfindung wird im Folgenden anhand eines Ausführungsbeispiels und mit Bezug auf die Zeichnungen näher erläutert. Es zeigen
- Fig. 1: eine Tabelle zum Ablauf eines erfindungsgemäßen Verfahrens und
- Fig. 2: eine Skizze zu einem Gerät zur Durchführung eines erfindungsgemäßen Verfahrens.

Die Ursache für die eingangs beschriebene mögliche Funktionsstörung an einem mit einem Aufbereitungsautomaten aufbereiteten ärztlichen bzw. dentalen Behandlungsinstrument wird darin gesehen, dass Reste des Reinigungsmittels im Bereich von Gleit- oder Lagerstellen im Inneren des Behandlungsinstruments eine geeignete Schmierung durch das ölhaltige Pflegemittel verhindern. Dies insbesondere, wenn das Reinigungsmittel Tenside beinhaltet. Selbst, wenn das Behandlungsinstrument - hier auch kurz als "Instrument" bezeichnet - nach dem Reinigungsschritt mit Druckluft ausgeblasen wird oder durch Wärmezufuhr getrocknet wird, können sich an den genannten Gleit- oder Lagerstellen noch Reste des Reinigungsmittels befinden, die einen anschließenden Aufbau eines geeigneten Schmierfilms durch das ölhaltige Pflegemittel beeinträchtigen. Dies trifft insbesondere zu, wenn sich die genannten Stellen in einem nicht trockenen Zustand befinden, also noch mit Wasser behaftet sind.

Zur Durchführung eines erfindungsgemäßen Verfahrens kann insbesondere ein Gerät vorgesehen sein, wie es als solches aus der eingangs erwähnten WO 2011/101396 A1 bekannt ist. Wie oben erwähnt, kann dieses Gerät - wie in Fig. 2 skizziert - insbesondere eine Kammer 61 aufweisen, in der an einer Kupplung 5 das aufzubereitende Instrument 100 angeordnet werden kann. Soweit nicht anders angegeben, kann das Gerät entsprechend der Darstellung in der WO 2011/101396 A1 gestaltet sein, auf deren Inhalt hier insoweit ausdrücklich verwiesen wird.

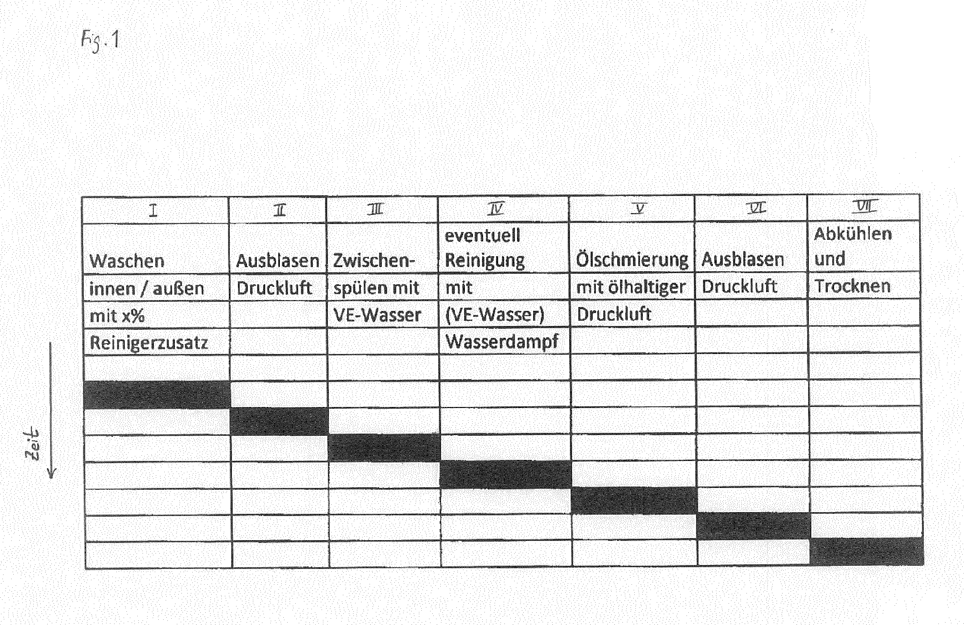

Erfindungsgemäß umfasst das Verfahren zum Reinigen und Pflegen eines entsprechenden Instruments 100 somit einen Reinigungsschritt, in der Tabelle der Fig. 1 mit "I" bezeichnet, in dem das Instrument mit Hilfe eines Reinigungsmittels gereinigt wird. Insbesondere eignet sich das Verfahren, wenn das Instrument 100 einen Innenraum aufweist, in dem sich wenigstens eine Gleit- oder Lagerstelle befindet. Eine solche Gleit- oder Lagerstelle kann beispielsweise Teil eines Spannsystems des Instruments 100 zur Halterung eines Behandlungswerkzeugs, beispielsweise eines Bohrers sein. Im Folgenden wird hier davon ausgegangen, dass mehrere solche Gleit- oder Lagerstellen im Instrumenteninneren vorhanden sind.

Bei dem Reinigungsschritt wird dabei dementsprechend vorzugsweise der Innenraum des Instruments mit dem Reinigungsmittel gereinigt, insbesondere die Gleit- oder Lagerstellen. Vorzugsweise wird dabei das Reinigungsmittel über die Kupplung 5 in den Innenraum geleitet.

Zusätzlich kann hierbei eine Reinigung einer äußeren Oberfläche des Instruments vorgesehen sein.

Insbesondere kann das Reinigungsmittel Tenside beinhalten.

In einem Spülschritt III, der zeitlich nach dem Reinigungsschritt I durchgeführt wird, wird das Reinigungsmittel zumindest im Wesentlichen vollständig entfernt, insbesondere aus dem Innenraum, insbesondere von den Gleit- oder Lagerstellen.

In diesem Spülschritt III erfolgt vorzugsweise ein Spülen des Instruments, insbesondere seines Innenraums mit Wasser, vorzugsweise mit VE-Wasser. Dabei kann vorgesehen sein, dass das Wasser über die Kupplung 5 zugeführt wird. Besonders bevorzugt wird hierbei Wasser verwendet, das keinerlei Zusatz aufweist. Hierdurch lässt sich das Reinigungsmittel besonders geeignet entfernen.

Beispielsweise kann dabei vorgesehen sein, dass in dem Spülschritt III das Spülen mit dem Wasser für mindestens 15 Sekunden durchgeführt wird.

In einem Öl-Schritt V, der zeitlich nach dem Spülschritt III durchgeführt wird, wird das Instrument, insbesondere dessen Innenraum, insbesondere die Gleit- oder Lagerstellen mit einem ölhaltigen Pflegemittel gepflegt. Vorzugsweise ist dabei vorgesehen, dass das ölhaltige Pflegemittel mithilfe von Druckluft in den Innenraum des Instruments bzw. an die Gleit- oder Lagerstellen transportiert wird, insbesondere über die Kupplung 5.

Vorzugsweise enthält das ölhaltige Pflegemittel einen Emulgator.

Vorzugsweise wird nach dem Spülschritt III und vor dem Öl-Schritt V das Instrument, insbesondere dessen Innenraum in einem Wasserdampfreinigungs-Schritt IV mit Wasserdampf gereinigt. Vorzugsweise handelt es sich hierbei um Dampf aus VE-Wasser; wiederum erfolgt vorzugsweise die Zufuhr des Dampfes über die Kupplung 5.

Durch den Wasserdampfreinigungs-Schritt IV lässt sich eine Erwärmung des Innenraums bzw. der Gleit- oder Lagerstellen bewirken, die dazu führt, dass beim folgenden Öl-Schritt das ölhaltige Pflegemittel besonders geeignet auf die Gleit- oder Lagerstellen aufgetragen wird.

Vorzugsweise wird nach dem Reinigungsschritt I und vor dem Spülschritt III das Reinigungsmittel in einem Ausblas-Schritt II mittels Druckluft aus bzw. von dem Instrument, insbesondere aus dem Innenraum des Instruments bzw. der Gleit- oder Lagerstelle teilweise entfernt. Hierdurch kann die Wirkung der in dem folgenden Spülschritt III erfolgenden Entfernung des Reinigungsmittels gesteigert werden. Vorzugsweise wird die Druckluft hierbei wieder entsprechend über die Kupplung 5 in den Innenraum geleitet.

Weiterhin kann vorteilhaft vorgesehen sein, dass nach dem Öl-Schritt V ein weiterer Ausblas-Schritt VI durch Zufuhr von Druckluft in den Innenraum des Instruments erfolgt, vorzugsweise über die Kupplung 5. Nach diesem weiteren Ausblas-Schritt VI kann dann noch ein Abkühl- und Trocken-Schritt durchgeführt werden.

## Patentansprüche

1. Verfahren zum Reinigen und Pflegen eines ärztlichen, insbesondere zahnärztlichen Behandlungsinstruments, bei dem das Instrument mit Hilfe eines Reinigungsmittels gereinigt und anschließend mittels einem ölhaltigen Pflegemittel gepflegt wird,
**dadurch gekennzeichnet,**
**dass** vor dem Zuführen des ölhaltigen Pflegemittels zunächst das Reinigungsmittel in einem Spülschritt im Wesentlichen vollständig entfernt wird.

2. Verfahren nach Anspruch 1,
**dadurch gekennzeichnet,**
**dass** in dem Spülschritt das Instrument mit Wasser, insbesondere mit VE-Wasser gespült wird.

3. Verfahren nach Anspruch 2,
**dadurch gekennzeichnet,**
**dass** nach dem Spülschritt vor einem Zuführen des ölhaltigen Pflegemittels das Instrument mit Wasserdampf gereinigt wird.

4. Verfahren nach einem der vorherigen Ansprüche,
**dadurch gekennzeichnet,**
**dass** nach dem Zuführen des Reinigungsmittels dieses vor dem Spülschritt zunächst mittels Druckluft aus bzw. von dem Instrument wenigstens teilweise entfernt wird.

5. Verfahren nach einem der vorherigen Ansprüche,
**dadurch gekennzeichnet,**
**dass** das ölhaltige Pflegemittel einen Emulgator enthält.
